**Europäisches Patentamt**

**European Patent Office**

**Office européen des brevets**

(19)

(11) Veröffentlichungsnummer: **0 358 107**
**A2**

(12) **EUROPÄISCHE PATENTANMELDUNG**

(21) Anmeldenummer: **89116068.1**

(22) Anmeldetag: **31.08.89**

(51) Int. Cl.⁵: **A61K 9/20 , A61J 3/10**

(30) Priorität: **07.09.88 DE 3830355**

(43) Veröffentlichungstag der Anmeldung:
**14.03.90 Patentblatt 90/11**

(84) Benannte Vertragsstaaten:
**DE FR GB IT**

(71) Anmelder: **BASF Aktiengesellschaft**
**Carl-Bosch-Strasse 38**
**D-6700 Ludwigshafen(DE)**

(72) Erfinder: **Klimesch, Roger, Dr.**
**Georg-Froeba-Strasse 43**
**D-6146 Alsbach-Haehnlein 2(DE)**
Erfinder: **Mrosek, Walter Dr.**
**Bluetenweg 1**
**D-6719 Bobenheim am Berg(DE)**
Erfinder: **Bleckmann, Gerhard, Dr.**
**Giselherstrasse 9**
**D-6840 Lampertheim 5(DE)**
Erfinder: **Farwerck, Karl-Peter**
**Enzingerstrasse 32**
**D-6520 Worms 21(DE)**
Erfinder: **Sanner, Axel, Dr.**
**Lorscher Ring 2 c**
**D-6710 Frankenthal(DE)**
Erfinder: **Schlemmer, Lothar**
**Duisbergstrasse 1 a**
**D-6701 Maxdorf(DE)**

(54) Verfahren zur Herstellung von pharmazeutischen Tabletten.

(57) Verfahren zur Herstellung von pharmazeutischen Tabletten auf üblichen Tablettiermaschinen, wobei man eine mindestens ein polymeres Bindemittel enthaltende pharmazeutische Mischung extrudiert und das noch plastische Material in einer konventionellen Tablettiermaschine zu festen pharmazeutischen Formlingen verarbeitet, sowie die entsprechende Vorrichtung.

EP 0 358 107 A2

## Verfahren zur Herstellung von pharmazeutischen Tabletten

Die Erfindung betrifft ein Verfahren zur Herstellung von pharmazeutischen Tabletten durch Extrusion einer mindestens ein polymeres Bindemittel enthaltenden pharmazeutischen Mischung und Verformen des erhaltenen plastischen bis zähflüssigen Materials auf einer konventionellen Tablettierpresse.

Es ist bekannt, daß man pharmazeutische Wirkstoffe enthaltende Polymerschmelzen extrudieren und durch Kalandrieren verformen kann (EP 240 906). Die Produktionsgeschwindigkeit ist dabei begrenzt, weil die Walzen sich nur entlang einer Linie berühren, so daß die Formen nie vollständig geschlossen sind, wodurch die Herstellung von Tabletten mit symmetrischer Form schwierig ist.

Beim Tablettieren auf den üblichen Tablettiermaschinen stellt sich dieses Problem nicht, da die Formen während der Verpressung vollständig geschlossen sind. Das übliche Verfahren erfordert intensiv vorgemischte und in komplizierter Weise granulierte Tablettiermassen und ist daher insgesamt mehrstufig und aufwendig.

Der Erfindung lag die Aufgabe zugrunde, das herkömmliche Tablettierverfahren zu vereinfachen.

Die Lösung dieser Aufgabe steht in dem Verfahren nach den Ansprüchen 1 bis 12.

Wenn auch Fälle denkbar sind, in denen ein Vormischen zweckmäßig ist, so daß ein einfacher Extruder genügt, ist es doch in der Regel wesentlich vorteilhafter, wenn der Extruder in üblicher Weise als ein- oder mehrwelliger Mischextruder gestaltet ist, so daß ein Vormischen nicht erforderlich ist. Der Mischextruder kann mehrere Einfüllstutzen, gegebenenfalls für die getrennte Zugabe von festen und flüssigen Mischungsbestandteilen, sowie einen Rohrstutzen zur Inertbegasung (in der Regel Stickstoff) und/oder Entgasung besitzen. Zur Erhöhung des Durchsatzes kann der Extruder mehr als eine Düse haben.

Die Formgebung schließt sich unmittelbar an den Extrusionsvorgang an, indem die heiße, noch plastische oder zähflüssige Masse auf konventionellen Tablettiermaschinen zu bei 25° C festen pharmazeutischen Formlingen verpreßt wird. Hierzu kann die Schmelze mit Hilfe eines gegebenenfalls beheizten Füllschuhes in die Matrize einer Tablettiermaschine gefüllt und anschließend unter Kühlung in an sich üblicher Weise verpreßt werden. Man kann die heiße, noch plastische oder zähflüssige Masse aber auch mit Hilfe von Düsen und-Dosiereinrichtungen direkt in die Matrizen der Tablettiermaschinen dosieren. Anschließend wird wiederum unter Kühlung in üblicher Weise verpreßt.

Eine weitere Möglichkeit besteht darin, den durch Extrusion erhaltenen plastischen Strang mit Hilfe von zwei gegenläufigen, mit Messern besetzten Walzen oder einer solchen Walze und einer festen Gegenschneide oder mit Hilfe zweier mit äquidistanten Messern besetzter gegenläufiger Bänder oder eines solchen Bandes und eines mitlaufenden glatten Bandes bzw. einer festen, ebenen Gegenfläche in volumengleiche Stücke mit erstarrter Oberfläche, aber noch plastisch verformbarem Kern zu zerschneiden und diese Stücke in die Matrizen einer üblichen Tablettiermaschine in an sich üblicher Weise einzulegen und zu verformen.

Übliche Tablettiermaschinen sind z.B. mit Ober- und Unterstempeln in Matrizen arbeitende Tablettierpressen, die als Rundläufer oder Exzenterpresse ausgeführt sein können. Temperiert wird in üblicher Art und Weise.

Feste pharmazeutische Formlinge sind Tabletten aller denkbaren Formen, Drageekerne, Kerne für Filmtabletten, Mikrotabletten für die Kapselabfüllung, jedoch keine Zäpfchen.

Die Vorteile des Verfahrens bestehen u.a. darin, daß es kontinuierlich arbeitet, so daß die normalerweise separat durchgeführten vorgeschalteten Schritte wie Einwiegen, Mischen, Granulieren, Trocknen, Sieben, noch einmal Mischen, Transportieren, Lagern und die Gefahr des Entmischens dabei entfallen. Außerdem entfällt die Notwendigkeit der gesonderten Zugabe von Fließ-, Schmier- und Trennmitteln. Das Verfahren ist - z.B. mit Hilfe von elektronischen Differential-Dosierwaagen mit Schneckenförderern - vollautomatisierbar, es ist daher kostengünstig und gut reproduzierbar. Die Zahl der Kontrollanalysen kann wesentlich reduziert werden.

Extrudierbare pharmazeutische Mischungen sind Mischungen mindestens eines pharmazeutischen Wirkstoffes mit mindestens einem für die Herstellung pharmazeutischer Tabletten üblichen Hilfsstoff, die durch Schmelzen oder Erweichen mindestens einer Komponente teigig bis zähflüssig und daher extrudierbar sind. Das sind insbesondere Mischungen, die pharmakologisch akzeptable Polymere enthalten (wobei die Glastemperatur der Mischung unter der Zersetzungstemperatur aller Mischungskomponenten liegt), beispielsweise Polyvinylpyrrolidon (PVP), Copolymerisate von N-Vinylpyrrolidon (NVP) und Vinylacetat, Copolymerisate von Vinylacetat und Crotonsäure, teilverseiftes Polyvinylacetat, Polyvinylalkohol, Ethylen/VinylacetatCopolymerisate, Polyhydroxyethylmethacrylat, Copolymerisate von Methylmethacrylat und Acrylsäure, Celluloseester, Celluloseether, Polyethylenglykol, Polyethylen. Die K-Werte (nach H. Fikentscher, Cellulose-Chemie 13 (1932), Seiten 58 bis 64 und 71 und 74) der Polymeren liegen im Bereich von 10 bis 100, vorzugsweise 12 bis 70, insbesondere 12 bis 35, für PVP vorzugsweise bei 12 bis 35, insbesondere bei 12 bis 17.

Das polymere Bindemittel muß in der Gesamtmischung aller Komponenten im Bereich von 50 bis 180, vorzugsweise 60 bis 130°C erweichen oder schmelzen, so daß die Masse extrudierbar ist. Die Glasübergangstemperatur der Mischung muß also auf jeden Fall unter 180, vorzugsweise unter 130°C liegen. Erforderlichenfalls wird sie durch übliche pharmakologisch akzeptable weichmachende Hilfsstoffe wie langkettige Alkohole, Ethylenglykol, Propylenglykol, Trimethylolpropan, Triethylenglykol, Butandiole, Pentanole, Hexanole, Polyethylenglykole, Silicone, aromatische Carbonsäureester (z.B. Dialkylphthalate, Trimelithsäureester, Benzoesäureester, Terephthalsäureester) oder aliphatische Dicarbonsäureester (z.B. Dialkyladipate, Sebacinsäureester, Azelainsäureester, Zitronen- und Weinsäureester) oder Fettsäureester herabgesetzt.

Bevorzugt werden NVP-Polymerisate, die in Mischung mit dem Wirkstoff und ggf. üblichen galenischen Hilfsstoffen mit oder vorzugsweise ohne weichmachende Zusätze im gewünschten Temperaturbereich schmelzen oder erweichen. Das Schmelzen oder Erweichen unterhalb einer bestimmten Temperatur ist gegebenenfalls erforderlich im Hinblick auf eine mögliche thermische und/oder oxidative Schädigung nicht nur des Wirkstoffs, sondern auch des NVP-Polymerisates. Dieses könnte beim Extrudieren vergilben, weshalb die Extrusion von NVP-Polymerisaten bisher nicht üblich ist. Die Gefahr ist jedoch gering bei Extrusionstemperaturen unter 180°C, vor allem unter 130°C, wenn das Polymerisat nicht in wäßriger Lösung mit Wasserstoffperoxid als Starter hergestellt wurde, sondern in einem organischen Lösungsmittel, oder aber in Wasser mit einem organischen Peroxid als Starter, etwa nach dem Verfahren gemäß EP-A-273 238 oder nach dem Verfahren von US 4 520 179 und 4 520 180.

Falls der K-Wert über 17, insbesondere über 30 oder gar 40 (bis maximal 70) liegt und keine stark weichmachende Komponente zugegen ist, kommen als NVP-Polymerisate nur solche mit einer Glastemperatur Tg unter 120, vorzugsweise unter 100°C in Betracht, oder das NVP-Polymerisat (einschließlich Homopolymer) darf nicht in Wasser mit $H_2O_2$ als Starter hergestellt worden sein. Dabei würden nämlich Polymer-Endgruppen entstehen, die bei höherer Temperatur zur Vergilbung führen.

Das NVP-Polymerisat kann über die Art und Menge an Comonomeren je nach Anwendungszweck so stark oder so schwach hydrophil eingestellt werden, daß sich die daraus hergestellten Tabletten im Mund (Buccaltablette) oder im Magen oder auch erst im Darm (rasch oder verzögert) auflösen oder so quellen, daß sie den Wirkstoff freigeben. Die Polymerisate sind dann ausreichend quellbar, wenn sie bei Lagerung bei 90 % relativer Luftfeuchtigkeit mehr als 10 Gew.% Wasser aufnehmen. Falls es bei carboxylgruppenhaltigen Bindemitteln erwünscht ist, daß sie erst im alkalischen Milieu des Darms den Wirkstoff freigeben, gilt die obige Angabe der Wasseraufnahme nur für die neutralisierte Form (Salzform) des Polymeren (in der die Protonen der Carboxylgruppen ganz oder teilweise durch Ammonium-, Natrium-oder Kaliumionen ersetzt sind).

Als Comonomer zum NVP kommen in Betracht: ungesättigte Carbonsäuren, z.B. Methacrylsäure, Grotonsäure, Maleinsäure, Itaconsäure, sowie deren Ester mit Alkoholen mit 1 bis 12, vorzugsweise 1 bis 8 Kohlenstoffatomen, ferner Hydroxyethyl- oder Hydroxypropylacrylat und -methacrylat, (Meth)acrylamid, die Anhydride und Halbester der Maleinsäure- und Itaconsäure (wobei der Halbester vorzugsweise erst nach der Polymerisation gebildet wird), N-Vinylcaprolactam und Vinylpropionat. Bevorzugte Comonomere sind Acrylsäure und insbesondere Vinylacetat. Es werden daher NVP-Polymerisate bevorzugt, die entweder nur NVP oder Vinylacetat als einziges Comonomeres einpolymerisiert enthalten. Vinylacetat und Vinylpropionat können nach der Polymerisation ganz oder teilweise verseift sein.

Übliche galenische Hilfsstoffe, deren Gesamtmenge bis zu 100 Gew.%, bezogen auf das Polymerisat, betragen kann, sind z.B. Streckmittel wie Silikate oder Kieselerde, Stearinsäure oder deren Salze mit z.B. Magnesium oder Kalzium, Methylcellulose, Natrium-Carboxymethylcellulose, Talkum, Saccharose, Lactose, Getreide- oder Maisstärke, Kartoffelmehl, Polyvinylalkohol, ferner Netz-, Konservierungs-, Spreng-, Adsorptionsmittel, Farbstoffe, Geschmacksstoffe (vgl. z.B. H. Sucker et al., Pharmazeutische Technologie, Thieme-Verlag, Stuttgart 1978).

Falls gewünscht, kann die erfindungsgemäß hergestellte Tablette auch mit einem üblichen Überzug zur Verbesserung des Aussehens und/oder des Geschmacks (Dragee, Filmtablette) oder zwecks zusätzlicher Verzögerung der Wirkstofffreigabe versehen werden. Für oral einzunehmende Tabletten mit verzögerter Wirkstofffreisetzung kann es günstig sein, wenn man die Tablette nach einer der bekannten Techniken in geschlossenzellig poröser Form herstellt, damit sie im Magen aufschwimmt und dadurch länger dort verweilt. Ferner können nach dem erfindungsgemäßen Verfahren sehr kleine Tabletten hergestellt werden, die mit Vorteil anstelle herkömmlicher Granulate in Kapseln abgefüllt werden. Der Begriff "Tablette" im Sinne der Erfindung ist weder an eine bestimmte Form noch an die perorale Anwendung gebunden. Er schließt vielmehr auch (nicht bei Körpertemperatur schmelzende) Zäpfchen zur rektalen Anwendung ein.

3

Unter pharmazeutischen Wirkstoffen im Sinne der Erfindung sind alle Stoffe mit einer pharmazeutischen Wirkung und möglichst geringen Nebenwirkungen zu verstehen, sofern sie sich unter den Verarbeitungsbedingungen nicht zersetzen. Die Wirkstoffmenge pro Dosiseinheit und die Konzentration können je nach Wirksamkeit und Freisetzungsgeschwindigkeit in weiten Grenzen variieren. Die einzige Bedingung ist, daß sie zur Erzielung der gewünschten Wirkung ausreichen. So kann die Wirkstoffkonzentration im Bereich von 0,1 bis 95, vorzugsweise von 20 bis 80, insbesondere 30 bis 70 Gew.% liegen. Auch Wirkstoff-Kombinationen können eingesetzt werden. Wirkstoffe im Sinne der Erfindung sind auch Vitamine.

Das erfindungsgemäße Verfahren ist beispielsweise zur Verarbeitung folgender Wirkstoffe geeignet:

Betamethason, Thioctsäure, Sotalol, Salbutamol, Norfenefrin, Silymarin, Dihydergotamin, Buflomedil, Etofibrrat, Indometacin, Oxazepam, ß-Acetyldigoxin, Piroxicam, Haloperidol, ISMN, Amitriptylin, Diclofenac, Nifedipin, Verapamil, Pyritinol, Nitrendipin, Doxycyclin, Bromhexin, Methylprednisolon, Glonidin, Fenofibrat, Allopurinol, Pirenzepin, Levothyroxin, Tamoxifen, Metildigoxin, o-(ß-Hydroxyethyl)-rutosid, Propicillin, Aciclo-virmononitrat, Paracetamol, Naftidrofuryl, Pentoxifyllin, Propafenon, Acebutolol, L-Thyroxin, Tramadol, Bromocriptin, Loperamid, Ketotifen, Fenoterol, Ca-Dobelisat, Propranolol, Minocyclin, Nicergolin, Ambroxol, Metoprolol, ß-Sitosterin, Enalaprilhydrogenmaleat, Bezafibrat, ISDN, Gallopamil, Xantinolnicotinat, Digitoxin, Flunitrazepan, Bencyclan, Dexapanthenol, Pindolol, Lorazepam, Diltiazem, Piracetam, Phenoxymethylpenicillin, Furosemid, Bromazepam, Flunarizin, Erythromycin, Metoclopramid, Acemetacin, Ranitidin, Biperiden, Metamizol, Doxepin, Dikalium-Chlorazepat, Tetrazepam, Estramustinphosphat, Terbutalin, Captopril, Maprotilin, Prazosin, Atenolol, Glibenclamid, Cefaclor, Etilefrin, Cimetidin, Theophyllin, Hydromorphon, Ibuprofen, Primidon, Clobazam, Oxaceprol, Medroxyprogesteron, Flecainid, Mg-Pyridoxal-5-phosphatglutaminat, Hymechromon, Etofyllinclofibrat, Vincamin, Cinnarizin, Diazepam, Ketoprofen, Flupentixol, Molsidomin, Glibornurid, Dimetinden, Melperon, Soquinolol, Dihydrocodein, Clomethiazol, Clemastin, Glisoxepid, Kallidinogenase, Oxyfedrin, Baclofen, Carboxymethylcystein, Thioridacin, Betahistin, L-Tryptophan, Myrtol, Bromelaine, Prenylamin, Salazosulfapyridin, Astemizol, Sulpirid, Benzerazid, Dibenzepin, Acetylsalicylsäure, Miconazol, Nystatin, Ketoconazol, Na-Picosulfat, Colestyramin, Gemfibrocil, Rifampicin, Fluorcortolon, Mexiletin, Amoxicillin, Terfenadrin, Mucopolysaccharidpolyschwefelsäureester, Triazolam, Mianserin, Tiaprofensäure, Ameziniummetilsulfat, Mefloquin, Probucol, Chinidin, Carbamazepin, Mg-L-aspartat, Penbutolol, Piretanid, Amitriptylin, Cyproteron, Na-Valproinat, Mebeverin, Bisacodyl, 5-Amino-Salicylsäure, Dihydralazin, Magaldrat, Phenprocoumon, Amantadin, Naproxen, Carteolol, Famotidin, Methyldopa, Auranofin, Estriol, Nadolol, Levomepromazin, Doxorubicin, Medofenoxat, Azathioprin, Flutamid, Norfloxacin, Fendilin, Prajmaliumbitartrat, Aescin.

Besonders bevorzugt werden feste Lösungen folgender Wirkstoffe: Acetaminophen (= Paracetamol), Acetohexamid, Acetyldigoxin, Acetylsalicylsäure, Acromycin, Anipamil, Benzocain, ß-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketoprofen, Lonetil, Medazepam, Mefrusid, Methandrostenolon, Methylprednisolon, Methylsulfadiazin (= Sulfaperin), Nalidixinsäure, Nifedipin, Nitrazepam, Nitrofurantoin, Nystatin, Östradiol, Papaverin, Phenacetin, Phenobarbital, Phenylbutazon, Phenytoin, Prednison, Reserpin, Spironolacton, Streptomycin, Sulfadimidin (= Sulfamethazin), Sulfamethizol, Sulfamethoxazol, Sulfamethoxydiazin (= Sulfameter), Sulfaperin, Sulfathiazol, Sulfisoxazol, Testosteron, Tolazamid, Tolbutamid, Trimethoprim, Tyrothricin.

Der Begriff "feste Lösungen" ist dem Fachmann geläufig, beispielsweise aus der eingangs zitierten Literatur. In festen Lösungen von pharmazeutischen Wirkstoffen in Polymeren liegt der Wirkstoff molekulardispers im Polymeren vor.

Die Bildung fester Lösungen der genannten Wirkstoffe in NVP-Polymerisaten war nicht vorherzusehen und ist umso überraschender, als viele in Wasser schwerlösliche Wirkstoffe in anderen Polymeren keine festen Lösungen (mit molekulardisperser Verteilung) bilden, sondern sich in Form fester Teilchen in das jeweilige Polymere einlagern, die elektronenmikroskopisch zu erkennen sind. Im Falle kristalliner Wirkstoffe zeigen sie auch ein Debye-Scherrer-Diagramm, im Gegensatz zu den festen Lösungen.

Falls zusätzlich zu dem erfindungsgemäß einzusetzenden noch weitere wasserlösliche, schmelzbare Bindemittel verwendet werden, soll das erstgenannte mindestens 50, vorzugsweise mindestens 70 Gew.% aller eingesetzten schmelzbaren Bindemittel ausmachen.

Die in den Beispielen genannten Teile und Prozente beziehen sich auf das Gewicht.

Beispiel 1

45 Teile eines Copolymerisats vom K-Wert 30 aus 60 Gew.% N-Vinylpyrrolidon und 40 Gew.%

4

Vinylacetat, 5 Teile Stearylalkohol und 50 Teile Theophyllin wurden in einem Doppelschneckenextruder vermischt und extrudiert. Die Temperatur des sechs Schüsse enthaltenden Extrudermantels betrug 30, 60, 60, 60, 80 und 100° C; die Düse wurde auf 100° C aufgeheizt. Der hierbei erhaltene Strang wurde direkt mit einer auf +15° C gekühlten Tablettenpresse zu Oblongtabletten verpreßt. Es entstanden starre Tabletten.

Die so erhaltenen Tabletten waren gegen mechanische Einflüsse stabil und zeigten keinen Abrieb beim Transport und Verpacken.

Beispiel 2

50 Teile des Copolymerisats von Beispiel 1 und 50 Teile Theophyllin wurden in einem Doppelschneckenextruder vermischt und extrudiert. Abweichend von Beispiel 1 wurde die Temperatur der Schüsse auf 30, 60, 60, 60, 90 und 120° C eingestellt. Die Düse hatte ebenfalls eine Temperatur von 120° C. Der erhaltene Strang wurde analog zu Beispiel 1 zu Oblongtabletten verpreßt (Temp. der Tablettenpresse: +15° C). Auch diese Tabletten waren gegen mechanische Einflüsse stabil.

Beispiel 3

47,5 Teile eines Copolymerisats vom K-Wert 30 aus 60 Gew.% N-Vinylpyrrolidon und 40 Gew.% Vinylacetat, 2,5 Teile vernetztes PVP als Tablettensprengmittel und 50 Teile Theophyllin wurden in einem Doppelschneckenextruder vermischt und extrudiert. Die Temperatur der fünf Schüsse betrug jeweils 120° C, die Düse hatte eine Temperatur von 130° C. Der noch plastische Strang wurde wie bei Beispiel 1 zu Oblongtabletten verpreßt. Die Tabletten waren stabil gegen mechanische Einflüsse (Temp. der Tablettenpresse: +15° C).

Beispiel 4

Teile eines Copolymerisats vom K-Wert 52 aus 30 Gew.% N-Vinylpyrrolidon und 70 Gew.% Vinylacetat und 50 Teile Theophyllin wurden in einem Doppelschneckenextruder gemischt und extrudiert. Die Temperatur der fünf Schüsse betrug 30, 60, 100, 100 und 120° C. Die Düse wurde ebenfalls auf 120° C aufgeheizt. Der noch plastische Strang wurde wie bei Beispiel 1 zu mechanisch stabilen Oblongtabletten verpreßt (Temp. der Tablettenpresse: +15° C).

Beispiele 5 bis 8

Eine Mischung von 50 Gew.% eines N-Vinylpyrrolidon-Homopolymeren (PVP) von dem jeweils in der Tabelle angegebenen K-Wert und 50 Gew.% Theophyllin wurden in einem Einwellen-Extruder bei der jeweils in der Tabelle angegebenen Temperatur aufgeschmolzen, extrudiert und wie bei Beispiel 1 zu Tabletten verformt.

| Beispiel | K-Wert | T [° C] | | | | | Düse | Temp. der Tablettenpresse [° C] |
|---|---|---|---|---|---|---|---|---|
| | | 1. | 2. | 3. | 4. | 5. | | |
| | | Schuß | | | | | | |
| 5 | 12 | 115 | 125 | 135 | 135 | 135 | 145 | 10 |
| 6 | 17 | 125 | 125 | 135 | 145 | 145 | 155 | 10 |
| 7 | 25 | 145 | 155 | 165 | 175 | 175 | 175 | 15 |
| 8 | 30 | 150 | 160 | 160 | 170 | 180 | 180 | 15 |
| 8a | 60 | 150 | 160 | 160 | 170 | 180 | 180 | 15 |

5

Beispiel 9

40 Teile eines Copolymerisats aus 60 Gew.% N-Vinylpyrrolidon und 40 Gew.% Vinylacetat vom K-Wert 30, 10 Teile Polyhydroxyethylmethacrylat und 50 Teile Theophyllin wurden analog zu Beispiel 1 zu mechanisch stabilen Tabletten verarbeitet. Temperatur der Schüsse: 70, 80, 80, 80, 80°C. Düse: 90°C. Tablettenpresse: +20°C.

Beispiel 10

50 Teile eines handelsüblichen, zu 80 verseiften Polyvinylacetats und 50 Teile Theophyllin wurden analog zu Beispiel 1 verarbeitet. Die Temperatur der 5 Schüsse betrug 100, 100, 110; 120, 130°C. Düse: 150°C. Tablettenpresse: +20°C.

Beispiel 11

50 Teile Polyhydroxyethylmethacrylat vom K-Wert 30 und 50 Teile Theophyllin wurden analog Beispiel 1 verarbeitet. Temperatur der Schüsse: 120, 130, 150, 160, 160°C. Düse: 170°C. Tablettenpresse: +32°C.

Beispiel 12 bis 14

36 Teile eines Copolymerisates aus 60 Gew.% N-Vinylpyrrolidon und 40 Gew.% Vinylacetat vom K-Wert 30, 4 Teile Stearylalkohol, 40 Teile Theophyllin und 20 Teile
Beispiel 12) Stärke
Beispiel 13) Lactose
Beispiel 14) Saccharose
wurden in einem 6-schüssigen Dopppelschneckenextruder gemischt und analog Beispiel 1 zu Tabletten verformt. Die Temperatur der Schüsse betrug 90, 100, 110, 120, 120, 130°C, die der Düse 135°C. Tablettenpresse: +15°C.

Beispiele 15 bis 17

50 Teile des Copolymerisates der Beispiele 12 bis 14 und 50 Teile Verapamil wurden gemäß den Beispielen 12 bis 14 zu Tabletten geformt.
Analog den obigen Beispielen wurden die folgenden durchgeführt. Die Bedingungen der Verarbeitung sowie die Freisetzungsgeschwindigkeiten beim half-change-Test (vgl. R. Voigt, Lehrbuch der pharmazeutischen Technologie, 5. Aufl., Verl. Chemie, Weinheim; Deerfield Beach, Florida; Basel, 1984, S. 627, in Verbindung mit der Paddle-Methode nach USP 21) sind tabellarisch erfaßt. Zur Formgebung wurde eine temperierbare Tablettenpresse verwendet.

Tabelle

| Beisp. Nr. | Wirkstoff | Poly-mer | Hilfs-stoff | Gew.-Verhältnis Wirkst./Polymer/ Hilfsstoff |
|---|---|---|---|---|
| 18 | Pseudoephedrin 47,5 Diphenhydramin 2,5 | A | ./. | 50/50/0 |
| 19 | Propafenon | A | Stärke | 40/40/20 |
| 20 | Propafenon | A | StA | 60/35/5 |
| 21 | Propafenon | A | StA | 60/30/10 |
| 22 | Propafenon | A | StS | 60/35/5 |
| 23 | Propafenon | B | StA | 50/40/10 |
| 24 | Propafenon | A | MgSt | 60/35/5 |
| 25 | Propafenon | A | MgSt | 50/40/10 |
| 26 | Anipamil | A | MgSt | 50/40/10 |
| 27 | Vitamin B1 | B | ./. | 50/50/0 |
| 28 | Nicotinsäure | A | ./. | 50/50/0 |
| 29 | Biperiden | A | StA | 50/45/5 |
| 30 | Biperiden | A | ./. | 50/50/0 |
| 31 | Canthaxantin | B | ./. | 50/50/0 |
| 32 | Canthaxantin | A | ./. | 50/50/0 |

A = Copolymer aus 60 Gew.% NVP und 40 Gew.% Vinylacetat, K-Wert ca. 33

B = PVP, K-Wert 12

StA = Stearylalkohol

StS = Stearinsäure

MgSt = Magnesiumstearat

EP 0 358 107 A2

Tabelle (Fortsetzung)

| Beisp. Nr. | T1 | T2 | T3 | T4 | T5 | T6 | Düse | Freisetzungs- geschwindig- keit | Temperatur der Tablettenpresse [°C] |
|---|---|---|---|---|---|---|---|---|---|
| 18 | 60 | 80 | 100 | 120 | 120 | 120 | 120 | 100 % in 1 h | 16 |
| 19 | 60 | 70 | 90 | 110 | 110 | 110 | 110 | 100 % in 1 h | 16 |
| 20 | 80 | 90 | 100 | 120 | 140 | 140 | 140 | 100 % in 2 h | 15 |
| 21 | 80 | 90 | 100 | 120 | 130 | 130 | 140 | 52 % in 6 h | 15 |
| 22 | 70 | 90 | 100 | 110 | 115 | 115 | 115 | 42 % in 6 h | 15 |
| 23 | 65 | 80 | 95 | 110 | 110 | 110 | 110 | 100 % in 6 h | 15 |
| 24 | 60 | 70 | 80 | 80 | 95 | 100 | 100 | 95 % in 6 h | 10 |
| 25 | 60 | 70 | 80 | 80 | 95 | 100 | 100 | 80 % in 6 h | 10 |
| 26 | 30 | 30 | 40 | 40 | 60 | 60 | 60 | 100 % in 2 h | 10 |
| 27 | 40 | 40 | 50 | 60 | 80 | 80 | 80 | 100 % in 1 h | 10 |
| 28 | 60 | 70 | 80 | 95 | 95 | 100 | 100 | 100 % in 1 h | 10 |
| 29 | 80 | 90 | 100 | 120 | 120 | 130 | 135 | 100 % in 4 h | 16 |
| 30 | 80 | 90 | 110 | 120 | 140 | 140 | 140 | 100 % in 1 h | 16 |
| 31 | 30 | 30 | 40 | 40 | 60 | 60 | 60 | 100 % in 1 h | 20 |
| 32 | 40 | 40 | 55 | 60 | 60 | 80 | 80 | 100 % in 1 h | 20 |

EP 0 358 107 A2

**Ansprüche**

1. verfahren zur Herstellung von pharmazeutischen Tabletten auf üblichen Tablettiermaschinen, dadurch gekennzeichnet, daß man eine mindestens ein polymeres Bindemittel enthaltende pharmazeutische Mischung extrudiert und das noch plastische Material in einer konventionellen Tablettiermaschine zu bei 25°C festen pharmazeutischen Formlingen verarbeitet.

2. Verfahren nach Anspruch 1, dadurch gekennzeichnet, daß man wirkstoffhaltige Schmelzen von N-Vinylpyrrolid-2-on (NVP)-Polymerisaten extrudiert und verpreßt.

3. Verfahren nach Anspruch 2, dadurch gekennzeichnet, daß man wirkstoffhaltige Schmelzen von NVP-Polymerisaten extrudiert und verpreßt, wobei das NVP-Polymerisat entweder in einem organischen Lösungsmittel oder mit einem organischen Peroxid in Wasser hergestellt wurde.

4. Verfahren nach Anspruch 2, dadurch gekennzeichnet, daß man ein polymeres Bindemittel einsetzt, das mindestens 60 Gew.% NVP einpolymerisiert enthält.

5. Verfahren nach Anspruch 2, dadurch gekennzeichnet, daß man ein polymeres Bindemittel einsetzt, das aus NVP besteht oder neben NVP nur Vinylacetat einpolymerisiert enthält.

6. Verfahren nach Anspruch 1, dadurch gekennzeichnet, daß man einen in Wasser schwer löslichen Wirkstoff einsetzt, der sich in der Polymerschmelze ohne Zusatz von Lösungsmitteln oder Wasser molekulardispers löst und nach dem Erstarren der Schmelze eine feste Lösung bildet.

7. Verfahren nach Anspruch 6, dadurch gekennzeichnet, daß man dazu mindestens einen Wirkstoff aus folgender Gruppe einsetzt: Acetaminophen ( = Paracetamol), Acetohexamid, Acetyldigoxin, Acetylsalicylsäure, Acromycin, Anipamil, Benzocain, β-Carotin, Chloramphenicol, Chlordiazepoxid, Chlormadinonacetat, Chlorothiazid, Cinnarizin, Clonazepam, Codein, Dexamethason, Diazepam, Dicumarol, Digitoxin, Digoxin, Dihydroergotamin, Drotaverin, Flunitrazepam, Furosemid, Gramicidin, Griseofulvin, Hexobarbital, Hydrochlorothiazid, Hydrocortison, Hydroflumethiazid, Indomethazin, Ketoprofen, Lonetil, Medazepam, Mefrusid, Methandrostenolon, Methylprednisolon, Methylsulfadiazin( = Sulfaperin), Nalidixinsäure, Nifedipin, Nitrazepam, Nitrofurantoin, Nystatin, Östradiol, Papaverin, Phenacetin, Phenobarbital, Phenyl butazon, Phenytoin, Prednison, Reserpin, Spironolacton, Streptomycin, Sulfadimidin ( = Sulfamethazin), Sulfamethizol, Sulfamethoxazol, Sulfamethoxydiazin ( = Sulfameter), Sulfaperin, Sulfathiazol, Sulfisoxazol, Testosteron, Tolazamid, Tolbutamid, Trimethoprim, Tyrothricin.

8. Vorrichtung zur Durchführung des Verfahrens nach Anspruch 1, bestehend aus einem Mischextruder mit einem oder mehreren Einfüllstutzen und mit einer Strangdüse sowie einer Tablettiermaschine mit gekühltem Matrizenteller, die statt des üblichen Füllschuhs eine Aufgabevorrichtung für das heiße, plastische bis zähflüssige Material besitzt, die aber im übrigen in üblicher Weise im Taktverfahren mit Ober- und Unterstempel arbeitet.

9. Vorrichtung nach Anspruch 8, dadurch gekennzeichnet, daß der Füllschuh beheizbar und gegen den Matrizenteller thermisch isoliert ist.

10. Vorrichtung zur Durchführung des Verfahrens nach Anspruch 1, bestehend aus einem Mischextruder mit einem oder mehreren Einfüllstutzen und mit einer Strangdüse zur Erzeugung eines plastischen Stranges, einer temperierbaren Messerwalze oder einem mit äquidistanten Messern versehenen temperierbaren Band zum Zerschneiden des Stranges in volumengleiche Stücke, und einer daran angeschlossenen konventionellen Tablettenpresse, die die volumengleichen Stücke in die endgültige Form preßt.